# EUROPEAN PATENT SPECIFICATION

(11) **EP 4 186 541 B1**
(45) Date of publication and mention of the grant of the patent: **11.02.2026**
(21) Application number: 21211465.6
(22) Date of filing: 30.11.2021
(51) Int. Cl.: A61M 5/00, A61M 5/145

(54) **A METHOD FOR PROVIDING A TEMPERATURE INFORMATION REGARDING A CONTRAST MEDIUM AND A CONTRAST MEDIA INJECTION SYSTEM**
VERFAHREN ZUR BEREITSTELLUNG VON TEMPERATURINFORMATIONEN BEZÜGLICH EINES KONTRASTMITTELS UND KONTRASTMITTELINJEKTIONSSYSTEM
PROCÉDÉ PERMETTANT DE FOURNIR UNE INFORMATION DE TEMPÉRATURE CONCERNANT UN MILIEU DE CONTRASTE ET SYSTÈME D'INJECTION DE MILIEU DE CONTRASTE

(43) Date of publication of application: 31.05.2023
(73) Proprietor: Siemens Healthineers AG, 91301 Forchheim (DE)
(72) Inventor: Gutjahr, Ralf, 90459 Nürnberg (DE)
(74) Representative: Siemens Healthineers Patent Attorneys

(56) References cited:
- EP-A1- 3 437 683
- WO-A1-2015/034104
- WO-A1-2017/114718

## Description

The invention relates in one aspect to a method for providing a temperature information regarding a contrast medium. The invention relates in further aspects to a contrast media injection system and a medical imaging system.

Contrast media can be used to detect, characterize, and assess biological structures for various modalities such as computed tomography (CT), magnetic resonance imaging (MRI), ultrasound imaging and projection X-ray imaging (including angiography and fluoroscopy). Contrast media are also used to evaluate diseases, to determine treatment response, to identify target structures during biopsy and/or for needle guidance.

Whilst contrast media found their way into clinical routine since decades, in particular in test bolus and bolus tracking procedures, the optimal use of contrast media still remains a challenge in many situations. The contrast medium injection protocol may be selected and/or adjusted based on the technical and/or clinical conditions. The technical conditions may relate to the hardware, in particular the scanner and/or the injector, and/or to the software. The correct preparation of the contrast medica injection is also important, in particular with regard to setting up the injector site, an entanglement of the tubing and prewarming the contrast medium.

The molecular structure (e.g., the material's osmolality) and the concentration of the contrast medium has an impact on the viscosity of the material. The viscosity is an important material property that influences the injectability of intravenous (IV) agents, especially through small-bore needles (for adult, but especially for pediatric patients) and (central) IV catheters, and through long and/or small diameter tubing.

To prevent patient harm (e.g., extravasation/paravasation) modern contrast injectors may comprise mechanisms that regulate injection flow rates in case a factory or user set pressure threshold is exceeded. However, such pressure thresholds are easily reached when the contrast medium is too viscose.

Some diagnostic questions require a high contrast enhancement which is directly proportional to the contrast concentration and the injection flow rate (especially for fast scans/new scanners). Since it is not desired to compromise on either, another way to decrease the materials viscosity is required. This can be accomplished by prewarming the contrast medium before being injected into the patient's blood vessel system, in particular venous or arterial system.

According to one example, bottles filled with contrast medium can be stored in dedicated heating chambers/cabinets. In this case, the medical staff loads the chamber with the contrast media bottle minutes or, ideally, hours before the contrast injection. This comes with drawbacks. First, bigger bottles need a long time until they heat up to body temperature. Second, the chamber has limited space which can lead to shortage of warmed contrast medium when needed most. However, once the tubing between the injector and the patient is established, time can pass until the actual diagnostic contrast injection can occur. In this situation even previously heated contrast medium can cool down and viscosity can thereby increase.

According to another example, a contrast injector system is used with a heatable cuff around the loaded contrast medium. If the heatable cuff is provided only for the loaded contrast media bottles but not for the loaded saline bottles, prewarmed contrast medium is cooled down and material properties are impaired during dual flow injection phases, i.e., injections where contrast medium and saline are mixed. Therefore the injection must follow quickly and/or a shorter tubing system is required in order to ensure precise contrast injections. To a certain extent, larger bore injection needles can be applied to cope with the increased viscosity of a cold contrast medium.

It is an objective of the present invention to improve the handling of a contrast medium for medical imaging examinations with regard to viscosity. This problem is solved by subject matter claimed independently. Further advantageous embodiments and additional advantageous features are described in the dependent claims and in the specification.

The invention relates in one aspect to a method for providing a temperature information regarding a contrast medium, the method comprising:
- Generating thermography data of an injection tubing containing contrast medium,
- Calculating a temperature information regarding the contrast medium based on the thermography data of the injection tubing, and
- Providing the temperature information regarding the contrast medium.

The thermography data may comprise, for example, at least one thermogram of the injection tubing. The at least one thermogram may comprise, for example, for each position of a plurality of positions along the injection tubing a respective temperature of the injection tubing at that position.

The temperature information regarding the contrast medium may be indicative, for example, of a temperature of the contrast medium, a distribution of temperatures of the contrast medium along the injection tubing and/or a development of the temperature or temperatures of the contrast medium over time.

In another aspect, a temperature gradient along the injection tubing is calculated based on the thermography data of the injection tubing, wherein the temperature information regarding the contrast medium is calculated based on the temperature gradient along the injection tubing.

The temperature gradient may be indicative, for example, of a difference between a temperature of the contrast medium at an end of the injection tubing that is connected to the injector and a temperature of the contrast medium at an end of the injection tubing that is connected to a patient.

In another aspect, the thermography data of the injection tubing comprise a plurality of chronologically consecutive thermograms of the injection tubing, wherein the temperature information regarding the contrast medium is calculated based on a temporal change across the plurality of chronologically consecutive thermograms of the injection tubing.

The temporal change across the plurality of chronologically consecutive thermograms of the injection tubing may be related, for example, to the development of the temperature or temperatures of the contrast medium over time and/or to the development of the temperature gradient along the injection tubing of the contrast medium over time.

In another aspect, a threshold regarding a temperature-related quantity of the contrast medium is received, wherein the temperature information regarding the contrast medium is calculated further based on the threshold regarding the temperature-related quantity of the contrast medium.

The temperature-related quantity of the contrast medium may be calculated, for example, based on the thermography data of the injection tubing, in particular.

The temperature-related quantity of the contrast medium may be, for example, a quantitative measure for the temperature or temperatures of the contrast medium and/or for the temperature gradient along the injection tubing and/or for the temporal change across the plurality of chronologically consecutive thermograms of the injection tubing. The quantitative measure may be, for example, a statistical quantitative measure, in particular an average, a minimum, a maximum, a percentile or a range.

The threshold regarding the temperature-related quantity of the contrast medium may be, for example, provided in form of a factory setting of the contrast media injection system and/or selected by a user of the contrast media injection system.

In another aspect, the temperature information regarding the contrast medium is indicative of a relative position of the temperature-related quantity of the contrast medium with respect to the threshold, for example, in form of a comparison result of a comparison of the temperature-related quantity of the contrast medium and the threshold.

In another aspect, an output signal is generated based on the temperature information regarding the contrast medium and transmitted to a user.

The output signal may be, for example, an optical and/or acoustic signal. The output signal may be, for example, a warning signal or a release signal. The warning signal may indicate, for example, that the temperature of the contrast medium falls below a respective threshold. The release signal may indicate, for example, that the temperature of the contrast medium exceeds a respective threshold.

In another aspect, a heating control signal is calculated based on the temperature information regarding the contrast medium, wherein a heating system for heating the contrast medium is controlled based on the heating control signal.

In another aspect, an imaging control signal is generated based on the temperature information regarding the contrast medium, wherein a medical imaging device is controlled based on the imaging control signal.

For example, a medical imaging protocol and/or a contrast medium injection protocol may be adjusted automatically based on the temperature information. For example, a starting of a medical imaging examination by the medical imaging device can be prevented automatically based on the imaging control signal. For example, a starting of a medical imaging examination by the medical imaging device can be enabled automatically based on the imaging control signal. For example, the temperature information may be incorporated automatically into a header portion of medical imaging data.

The invention relates in one further aspect to a contrast media injection system, comprising a contrast media injector, an injection tubing, a thermographic camera, and a data processing system, wherein the injection tubing is connected to the contrast media injector, is containing contrast medium and is located within the field of view of the thermographic camera, wherein the thermographic camera is configured for generating thermography data of the injection tubing, wherein the data processing system is configured for calculating a temperature information regarding the contrast medium based on the thermography data and for providing the temperature information regarding the contrast medium.

The thermographic camera may be, for example, temperature sensitive and/or configured for detecting a temperature of the injection tubing and/or a temperature of the contrast medium. The injection tubing may be further connected to a patient, in particular for intravenous injection of the contrast medium.

The thermographic camera may be, for example, a 2D thermographic camera or a 3D thermographic camera. The thermographic camera may be, for example, configured for generating optical image data of the injection tubing in addition to the thermography data of the injection tubing.

The data processing system may be further configured for locating and/or segmenting the injection tubing in the thermography data of the injection tubing and/or in the optical image data of the injection tubing. The temperature information regarding the contrast medium may be calculated, for example, further based on a result of the locating and/or the segmenting of the injection tubing.

In another aspect, the contrast media injection system is configured for performing the method for providing the temperature information regarding the contrast medium.

In another aspect, the contrast media injection system further comprises a heating system for heating the contrast medium and a temperature controller for generating a heating control signal based on the temperature information regarding the contrast medium.

For example, a control loop for automatically adjusting the temperature of the contrast medium may be formed based on the thermographic camera, the data processing system, the heating system and the method for providing the temperature information regarding the contrast medium.

In another aspect, the contrast medium is iodine-based.

According to another aspect, the contrast medium is based on a material selected from the group consisting of gold, yttrium, zirconium, gadolinium, tungsten and hafnium. In particular, a contrast medium may be used, the viscosity of which increases with decreasing temperature.

For example, the pressure on the contrast medium in the injection tubing, that is needed for injection the contrast medium into the patient, can be decreased by 20% by heating up the contrast medium from room temperature (20°C) to human body temperature (36°C).

The invention relates in one further aspect to a medical imaging system, comprising the contrast media injection system and a medical imaging device.

In another aspect, the medical imaging system further comprises a patient table for positioning a patient with respect to the medical imaging device, wherein the patient table is located within the field of view of the thermographic camera.

The thermographic camera may be attached, for example, to the ceiling of an examination room, in which the medical imaging device is located. The thermographic camera may be located, for example, above the patient table. A further thermographic camera may be used, for example, for a side view on the patient table, to detect regions of the injection tubing hidden from a view from top. The thermographic camera may be located in such a way that the field of view of the thermographic camera covers an examination area of the medical imaging device, in particular in form of an inner region of an opening for receiving the patient.

In another aspect, the medical imaging device is selected from the group consisting of a computed tomography device, a magnetic resonance imaging device, an ultrasound imaging device and a projection X-ray imaging device. In particular, the medical imaging device may be a computed tomography device.

The projection X-ray imaging device may be, for example, an angiography imaging device and/or a fluoroscopy imaging device.

Thus, a cold and therefore viscose contrast medium can be detected prior to its injection to a patient. A prior-to-scan surveillance and warning mechanism can be provided to avoid contrast injections with a cold and viscose contrast medium. Furthermore, more consistent results of the medical imaging examination can be facilitated since variations due to the material properties of the contrast medium can be reduced.

The medical imaging examination workflow can be improved, in particular by reducing the number of non-diagnostic scans caused by contrast medium viscosity issues.

Through the use of the thermographic camera mechanical interactions with the injection tubing for temperature measurements can be avoided, thereby leaving out mechanical damage and surface disinfection issues. The patient comfort can be increased, in particular by preventing the injection of contrast medium that has a different temperature to the patient's body and/or by avoiding non-diagnostic scans caused by contrast medium viscosity issues.

The data processing system can comprise, for example, at least one of a cloud-computing system, a distributed computing system, a computer network, a computer, a tablet computer, a smartphone or the like. The data processing system can comprise hardware and/or software. The hardware can be, for example, a processor system, a memory system and combinations thereof. The hardware can be configurable by the software and/or be operable by the software. Data processing for performing an action of a method may be carried out in the processor.

Data, in particular each of the thermography data of injection tubing and the threshold, can be received, for example, by receiving a signal that carries the data and/or by reading the data from a computer memory. Data, in particular the temperature information, can be provided, for example, by transmitting a signal that carries the data and/or by writing the data into a computer memory and/or by displaying the data on a display.

In the context of the present invention, the expression "based on" can in particular be understood as meaning "using, inter alia". In particular, wording according to which a first feature is calculated (or generated, determined etc.) based on a second feature does not preclude the possibility of the first feature being calculated (or generated, determined etc.) based on a third feature.

Reference is made to the fact that the described methods and the described systems are merely preferred example embodiments of the invention, and that the invention can be varied by a person skilled in the art, without departing from the scope of the invention as it is specified by the claims.

The invention will be illustrated below with reference to the accompanying figures using example embodiments. The illustration in the figures is schematic and highly simplified and not necessarily to scale.
Fig. 1 shows a medical imaging system.
Fig. 2 shows a flow chart for a method for providing a temperature information regarding a contrast medium.

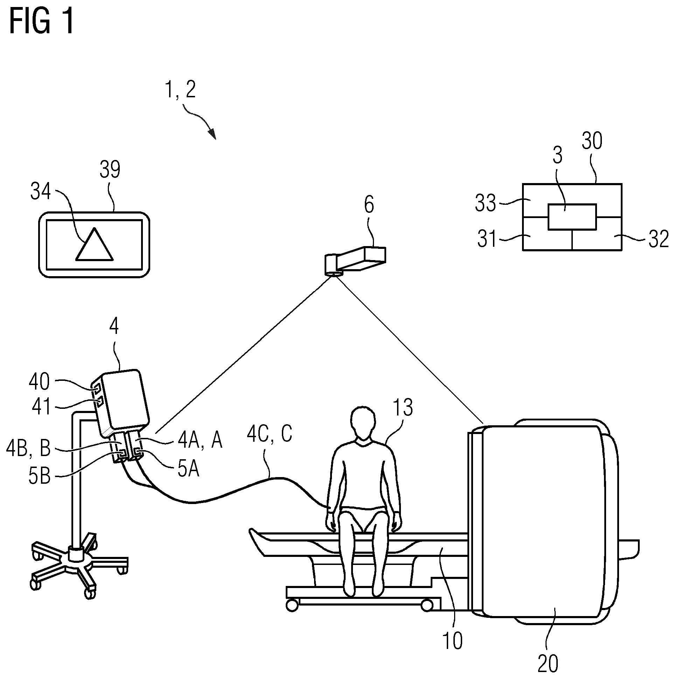

Fig. 1 shows the medical imaging system 2, comprising the contrast media injection system 1 and the medical imaging device 20.

The contrast media injection system 1 comprises the contrast media injector 4, an injection tubing 4C, a thermographic camera 6, and a data processing system 30,
- wherein the injection tubing 4C is connected to the contrast media injector 4, is containing contrast medium C and is located within the field of view of the thermographic camera 6,
- wherein the thermographic camera 6 is configured for generating thermography data of the injection tubing 4C,
- wherein the data processing system 30 is configured for calculating S2 a temperature information regarding the contrast medium C based on the thermography data and for providing S3 the temperature information regarding the contrast medium C.

The contrast media injection system 1 further comprises a heating system for heating the contrast medium C and a temperature controller 3 for generating a heating control signal based on the temperature information regarding the contrast medium C. The contrast medium C in this example is iodine-based.

The contrast media injector 4 comprises a first pressure syringe-plunger-pair 4A filled with the contrast medium reserve A and a second pressure syringe-plunger-pair 4B filled with saline reserve B. The contrast medium C in the injection tubing 4C can be an undiluted portion of the contrast medium reserve A or a mix of a portion of the contrast medium reserve A and a portion of the saline reserve B.

The contrast media injector 4 further comprises an interface 40 for receiving the heating control signal, for example, from the temperature controller 3, and a heating system for heating the contrast medium C. The heating system comprises a first heater 5A for heating the contrast medium reserve A and a second heater 5B for heating the saline reserve B. The contrast media injector 4 further comprises an interface 41 for receiving and/or providing contrast medium injection protocol data, for example to and/or from the data processing system 30. The data processing system 30 comprises a processor 30, a memory 31 and a data interface 32. The temperature controller 3 is implemented as a part of the data processing system 30.

The medical imaging system 2 further comprises a patient table 10 for positioning a patient 13 with respect to the medical imaging device 20, wherein the patient table 10 is located within the field of view of the thermographic camera 6.

The two thin lines emanating from the thermographic camera 6 schematically outline the field of view of the thermographic camera 6.

The medical imaging device 20 in this example is a computed tomography device or a magnetic resonance imaging device.

The medical imaging system 2 further comprises a touchsensitive display 39 for displaying the output signal 34 to a user and for receiving user inputs regarding the contrast media injection system 1 and the medical imaging device 20.

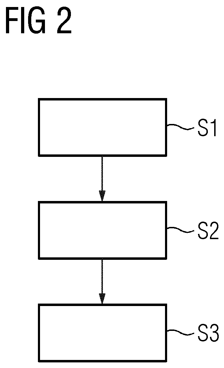

Fig. 2 shows a flow chart for a method for providing a temperature information regarding a contrast medium, the method comprising:
- Generating S1 thermography data of an injection tubing 4C containing contrast medium C,
- Calculating S2 a temperature information regarding the contrast medium C based on the thermography data of the injection tubing 4C,
- Providing S3 the temperature information regarding the contrast medium C.

An output signal 34 is generated based on the temperature information regarding the contrast medium C and transmitted to a user. A heating control signal is calculated based on the temperature information regarding the contrast medium C, wherein the heating system for heating the contrast medium C is controlled based on the heating control signal. An imaging control signal is generated based on the temperature information regarding the contrast medium C, wherein the medical imaging device 20 is controlled based on the imaging control signal.

## Claims

1. A method for controlling a heating system of a contrast media injection system (1), the contrast media injection system (1) comprising a contrast media injector (4), an injection tubing (4C), a thermographic camera (6), a data processing system (30), the heating system for heating a contrast medium (C) and a temperature controller (3) for generating a heating control signal based on a temperature information regarding the contrast medium (C), wherein the heating control signal is calculated based on the temperature information regarding the contrast medium (C), wherein the heating system for heating the contrast medium (C) is controlled based on the heating control signal, wherein the injection tubing (4C) is connected to the contrast media injector (4), is containing the contrast medium (C) and is located within the field of view of the thermographic camera (6), the method comprising:
- Generating (S1), by the thermographic camera (6), thermography data of the injection tubing (4C),
- Calculating (S2), by the data processing system (30), the temperature information regarding the contrast medium (C) based on the thermography data of the injection tubing (4C), wherein a temperature gradient along the injection tubing (4C) is calculated based on the thermography data of the injection tubing (4C), wherein the temperature information regarding the contrast medium (C) is calculated based on the temperature gradient along the injection tubing (4C), and
- Providing (S3), by the data processing system (30), the temperature information regarding the contrast medium (C).

2. The method according to claim 1,
- wherein the temperature gradient is indicative of a difference between a temperature of the contrast medium (C) at an end of the injection tubing (4C) that is connected to the contrast media injector (4) and a temperature of the contrast medium (C) at an end of the injection tubing (4C) that is connected to a patient (13).

3. The method according to claim 1 or 2,
- wherein the thermography data of the injection tubing (4C) comprise a plurality of chronologically consecutive thermograms of the injection tubing (4C),
- wherein the temperature information regarding the contrast medium (C) is calculated based on a temporal change across the plurality of chronologically consecutive thermograms of the injection tubing (4C).

4. The method according to one of the claims 1 to 3,
- wherein a threshold regarding a temperature-related quantity of the contrast medium (C) is received,
- wherein the temperature information regarding the contrast medium (C) is calculated further based on the threshold regarding the temperature-related quantity of the contrast medium (C).

5. The method according to claim 4,
- wherein the temperature information regarding the contrast medium (C) is indicative of a relative position of the temperature-related quantity of the contrast medium (C) with respect to the threshold.

6. The method according to one of the claims 1 to 5,
- wherein an output signal (34) is generated based on the temperature information regarding the contrast medium (C) and transmitted to a user.

7. The method according to one of the claims 1 to 6,
- wherein an imaging control signal is generated based on the temperature information regarding the contrast medium (C),
- wherein a medical imaging device (20) is controlled based on the imaging control signal.

8. A contrast media injection system (1), comprising a contrast media injector (4), an injection tubing (4C), a data processing system (30), a heating system for heating a contrast medium (C) and a temperature controller (3) for generating a heating control signal based on a temperature information regarding the contrast medium (C), wherein the heating control signal is calculated based on the temperature information regarding the contrast medium (C), wherein the heating system for heating the contrast medium (C) is controlled based on the heating control signal, wherein the injection tubing (4C) is connected to the contrast media injector (4) and is containing the contrast medium (C), and **characterised in that**
- the contrast media injector (4) comprises a thermographic camera (6), wherein the injection tubing (4C) is located within the field of view of the thermographic camera (6),
- wherein the thermographic camera (6) is configured for generating thermography data of the injection tubing (4C),
- wherein the data processing system (30) is configured for calculating (S2) the temperature information regarding the contrast medium (C) based on the thermography data and for providing (S3) the temperature information regarding the contrast medium (C), wherein a temperature gradient along the injection tubing (4C) is calculated based on the thermography data of the injection tubing (4C), wherein the temperature information regarding the contrast medium (C) is calculated based on the temperature gradient along the injection tubing (4C).

9. The contrast media injection system (1) according to claim 8, configured for performing a method according to one of the claims 1 to 7.

10. The contrast media injection system (1) according to claim 8 or 9,
- wherein the contrast medium (C) is iodine-based.

11. A medical imaging system (2), comprising
- the contrast media injection system (1) according to one of the claims 8 to 10,
- a medical imaging device (20).

12. The medical imaging system (2) according to claim 11,
- further comprising a patient table (10) for positioning a patient (13) with respect to the medical imaging device (20),
- wherein the patient table (10) is located within the field of view of the thermographic camera (6).

13. The medical imaging system (2) according to claim 11 or 12,
- wherein the medical imaging device (20) is selected from the group consisting of a computed tomography device, a magnetic resonance imaging device, an ultrasound imaging device and a projection X-ray imaging device.

## Patentansprüche

1. Verfahren zum Steuern eines Heizsystems eines Kontrastmittelinjektionssystems (1), wobei das Kontrastmittelinjektionssystem (1) einen Kontrastmittelinjektor (4), einen Injektionsschlauch (4C), eine thermografische Kamera (6), ein Datenverarbeitungssystem (30), das Heizsystem zum Erwärmen eines Kontrastmittels (C) und eine Temperatursteuerung (3) zum Generieren eines Heizsteuersignals basierend auf einer Temperaturinformation bezüglich des Kontrastmittels (C) umfasst, wobei das Heizsteuersignal basierend auf der Temperaturinformation bezüglich des Kontrastmittels (C) berechnet wird, wobei das Heizsystem zum Erwärmen des Kontrastmittels (C) basierend auf dem Heizsteuersignal gesteuert wird, wobei der Injektionsschlauch (4C) mit dem Kontrastmittelinjektor (4) verbunden ist, das Kontrastmittel (C) enthält und sich innerhalb des Sichtfelds der thermografischen Kamera (6) befindet, wobei das Verfahren Folgendes umfasst:
- Generieren (S1) von Thermografiedaten des Injektionsschlauchs (4C) durch die thermografische Kamera (6),
- Berechnen (S2) der Temperaturinformation bezüglich des Kontrastmittels (C) basierend auf den Thermografiedaten des Injektionsschlauchs (4C) durch das Datenverarbeitungssystem (30), wobei ein Temperaturgradient entlang des Injektionsschlauchs (4C) basierend auf den Thermografiedaten des Injektionsschlauchs (4C) berechnet wird, wobei die Temperaturinformation bezüglich des Kontrastmittels (C) basierend auf dem Temperaturgradienten entlang des Injektionsschlauchs (4C) berechnet wird, und
- Bereitstellen (S3) der Temperaturinformation bezüglich des Kontrastmittels (C) durch das Datenverarbeitungssystem (30).

2. Verfahren nach Anspruch 1,
- wobei der Temperaturgradient eine Differenz zwischen einer Temperatur des Kontrastmittels (C) an einem Ende des Injektionsschlauchs (4C), das mit dem Kontrastmittelinjektor (4) verbunden ist, und einer Temperatur des Kontrastmittels (C) an einem Ende des Injektionsschlauchs (4C), das mit einem Patienten (13) verbunden ist, angibt.

3. Verfahren nach Anspruch 1 oder 2,
- wobei die Thermografiedaten des Injektionsschlauchs (4C) eine Vielzahl von zeitlich aufeinanderfolgenden Thermogrammen des Injektionsschlauchs (4C) umfassen,
- wobei die Temperaturinformation bezüglich des Kontrastmittels (C) basierend auf einer zeitlichen Änderung über die Vielzahl von zeitlich aufeinanderfolgenden Thermogrammen des Injektionsschlauchs (4C) berechnet wird.

4. Verfahren nach einem der Ansprüche 1 bis 3,
- wobei eine Schwelle bezüglich einer temperaturbezogenen Menge des Kontrastmittels (C) empfangen wird,
- wobei die Temperaturinformation bezüglich des Kontrastmittels (C) ferner basierend auf der Schwelle bezüglich der temperaturbezogenen Menge des Kontrastmittels (C) berechnet wird.

5. Verfahren nach Anspruch 4,
- wobei die Temperaturinformation bezüglich des Kontrastmittels (C) eine relative Position der temperaturbezogenen Menge des Kontrastmittels (C) bezüglich der Schwelle angibt.

6. Verfahren nach einem der Ansprüche 1 bis 5,
- wobei ein Ausgabesignal (34) basierend auf der Temperaturinformation bezüglich des Kontrastmittels (C) generiert und an einen Benutzer übertragen wird.

7. Verfahren nach einem der Ansprüche 1 bis 6,
- wobei ein Bildgebungssteuersignal basierend auf der Temperaturinformation bezüglich des Kontrastmittels (C) generiert wird,
- wobei eine medizinische Bildgebungsvorrichtung (20) basierend auf dem Bildgebungssteuersignal gesteuert wird.

8. Kontrastmittelinjektionssystem (1), umfassend einen Kontrastmittelinjektor (4), einen Injektionsschlauch (4C), ein Datenverarbeitungssystem (30), ein Heizsystem zum Erwärmen eines Kontrastmittels (C) und eine Temperatursteuerung (3) zum Generieren eines Heizsteuersignals basierend auf einer Temperaturinformation bezüglich des Kontrastmittels (C), wobei das Heizsteuersignal basierend auf der Temperaturinformation bezüglich des Kontrastmittels (C) berechnet wird, wobei das Heizsystem zum Erwärmen des Kontrastmittels (C) basierend auf dem Heizsteuersignal gesteuert wird, wobei der Injektionsschlauch (4C) mit dem Kontrastmittelinjektor (4) verbunden ist und das Kontrastmittel (C) enthält, und **dadurch gekennzeichnet, dass**
- der Kontrastmittelinjektor (4) eine thermografische Kamera (6) umfasst, wobei sich der Injektionsschlauch (4C) innerhalb des Sichtfelds der thermografischen Kamera (6) befindet,
- wobei die thermografische Kamera (6) zum Generieren von Thermografiedaten des Injektionsschlauchs (4C) ausgelegt ist,
- wobei das Datenverarbeitungssystem (30) zum Berechnen (S2) der Temperaturinformation bezüglich des Kontrastmittels (C) basierend auf den Thermografiedaten und zum Bereitstellen (S3) der Temperaturinformation bezüglich des Kontrastmittels (C) ausgelegt ist, wobei ein Temperaturgradient entlang des Injektionsschlauchs (4C) basierend auf den Thermografiedaten des Injektionsschlauchs (4C) berechnet wird, wobei die Temperaturinformation bezüglich des Kontrastmittels (C) basierend auf dem Temperaturgradient entlang des Injektionsschlauchs (4C) berechnet wird.

9. Kontrastmittelinjektionssystem (1) nach Anspruch 8, das zum Durchführen eines Verfahrens nach einem der Ansprüche 1 bis 7 ausgelegt ist.

10. Kontrastmittelinjektionssystem (1) nach Anspruch 8 oder 9,
- wobei das Kontrastmittel (C) iodbasiert ist.

11. Medizinisches Bildgebungssystem (2), umfassend:
- das Kontrastmittelinjektionssystem (1) nach einem der Ansprüche 8 bis 10,
- eine medizinische Bildgebungsvorrichtung (20).

12. Medizinisches Bildgebungssystem (2) nach Anspruch 11,
- ferner umfassend einen Patiententisch (10) zum Positionieren eines Patienten (13) in Bezug auf die medizinische Bildgebungsvorrichtung (20),
- wobei sich der Patiententisch (10) innerhalb des Sichtfeldes der thermografischen Kamera (6) befindet.

13. Medizinisches Bildgebungssystem (2) nach Anspruch 11 oder 12,
- wobei die medizinische Bildgebungsvorrichtung (20) aus der Gruppe bestehend aus einer Computertomographievorrichtung, einer Magnetresonanzbildgebungsvorrichtung, einer Ultraschallbildgebungsvorrichtung und einer Projektionsröntgenbildgebungsvorrichtung ausgewählt ist.

## Revendications

1. Un procédé de commande d'un système de chauffage d'un système (1) d'injection d'agent de contraste, le système (1) d'injection d'agent de contraste comprenant un injecteur (4) d'agent de contraste, une tubulure (4C) d'injection, une caméra (6) thermographique, un système (30) de traitement de données, le système de chauffage pour chauffer un agent (C) de contraste et une unité (3) de commande de la température pour créer un signal de commande de chauffage sur la base d'une information de température concernant l'agent (C) de contraste, dans lequel on calcule le signal de commande de chauffage sur la base de l'information de température concernant l'agent (C) de contraste, dans lequel on commande le système de chauffage pour chauffer l'agent (C) de contraste sur la base du signal de commande de chauffage, dans lequel la tubulure (4C) d'injection communique avec l'injecteur (4) d'agent de contraste, contient l'agent (C) de contraste et est située dans le champ de vue de la caméra (6) thermographique, le procédé comprenant :
- créer (S1), par la caméra (6) thermographique, des données de thermographie de la tubulure (4C) d'injection,
- calculer (S2), par le système (30) de traitement de données, l'information de température concernant l'agent (C) de contraste sur la base des données de thermographie de la tubulure (4C) d'injection, dans lequel on calcule un gradient de température le long de la tubulure (4C) d'injection sur la base des données de thermographie de la tubulure (4C) d'injection, dans lequel on calcule l'information de température concernant l'agent (C) de contraste sur la base du gradient de température le long de la tubulure (4C) d'injection, et
- donner (S3), par le système (30) de traitement de données, l'information de température concernant l'agent (C) de contraste.

2. Le procédé suivant la revendication 1,
- dans lequel le gradient de température indique une différence entre une température de l'agent (C) de contraste à un bout de la tubulure (4C) d'injection, qui communique avec l'injecteur (4) d'agent de contraste et une température de l'agent (C) de contraste à un bout de la tubulure (4C) d'injection, qui communique avec un patient (13).

3. Le procédé suivant la revendication 1 ou 2,
- dans lequel les données de thermographie de la tubulure (4C) d'injection comprennent une pluralité de thermogrammes consécutifs chronologiquement de la tubulure (4C) d'injection,
- dans lequel on calcule l'information de température concernant l'agent (C) de contraste sur la base d'un changement temporel dans la pluralité de thermogrammes consécutifs chronologiquement de la tubulure (4C) d'injection.

4. Le procédé suivant l'une des revendications 1 à 3,
- dans lequel on reçoit un seuil concernant une quantité en relation avec la température de l'agent (C) de contraste,
- dans lequel on calcule en outre l'information de température concernant l'agent (C) de contraste sur la base du seuil concernant la quantité en relation avec la température de l'agent (C) de contraste.

5. Le procédé suivant la revendication 4,
- dans lequel l'information de température concernant l'agent (C) de contraste indique une position relative de la quantité en relation avec la température de l'agent (C) de contraste par rapport au seuil.

6. Le procédé suivant l'une des revendications 1 à 5,
- dans lequel on crée un signal (34) de sortie sur la base de l'information de température concernant l'agent (C) de contraste et on le transmet à un utilisateur.

7. Le procédé suivant l'une des revendications 1 à 6,
- dans lequel on crée un signal de commande d'imagerie sur la base de l'information de température concernant l'agent (C) de contraste,
- dans lequel on commande un dispositif (20) d'imagerie médicale sur la base du signal de commande d'imagerie.

8. Un système (1) d'injection d'agent de contraste, comprenant un injecteur (4) d'agent de contraste, une tubulure (4C) d'injection, un système (30) de traitement de données, un système de chauffage pour chauffer un agent (C) de contraste et une unité (3) de commande de la température pour créer un signal de commande de chauffage sur la base d'une information de température concernant l'agent (C) de contraste, dans lequel le signal de commande de chauffage est calculé sur la base de l'information de température concernant l'agent (C) de contraste, dans lequel le système de chauffage pour chauffer l'agent (C) de contraste est commandé sur la base du signal de commande de chauffage, dans lequel la tubulure (4C) d'injection communique avec l'injecteur (4) d'agent de contraste et contient l'agent (C) de contraste, et **caractérisé en ce que**
- l'injecteur (4) d'agent de contraste comprend une caméra (6) thermographique, dans lequel la tubulure (4C) d'injection est placé dans le champ de vue de la caméra (6) thermographique,
- dans lequel la caméra (6) thermographique est configurée pour donner des données de thermographie de la tubulure (4C) d'injection,
- dans lequel le système (30) de traitement de données est configuré pour calculer (S2) l'information de température concernant l'agent (C) de contraste sur la base des données de thermographie et pour donner (S3) l'information de température concernant l'agent (C) de contraste, dans lequel un gradient de température le long de la tubulure (4C) d'injection est calculée sur la base des données de thermographie de la tubulure (4C) d'injection, dans lequel l'information de température concernant l'agent (C) de contraste est calculée sur la base du gradient de température le long de la tubulure (4C) d'injection.

9. Le système (1) d'injection d'agent de contraste suivant la revendication 8, configuré pour effectuer un procédé suivant l'une des revendications 1 à 7.

10. Le système (1) d'injection d'agent de contraste suivant la revendication 8 ou 9,
- dans lequel l'agent (C) de contraste est iodé.

11. Le système (2) d'imagerie médicale, comprenant
- le système (1) d'injection d'agent de contraste de l'une des revendications 8 à 10,
- un dispositif (20) d'imagerie médicale.

12. Le système (2) d'imagerie médicale suivant la revendication 11,
- comprenant en outre une table (10) de patient pour mettre un patient (13) en position par rapport au dispositif (20) d'imagerie médicale,
- dans lequel la table (10) de patient est placée dans le champ de vue de la caméra (6) thermographique.

13. Le système (2) d'imagerie médicale suivant la revendication 11 ou 12,
- dans lequel le dispositif (20) d'imagerie médicale est choisi dans le groupe consistant en un dispositif de tomodensitométrie assistée par ordinateur, un dispositif d'imagerie par résonnance magnétique, un dispositif d'imagerie aux ultrasons et un dispositif d'imagerie par rayons X par projection.
